# EUROPEAN PATENT APPLICATION

(11) **EP 4 537 757 A1**
(43) Date of publication of application: **16.04.2025**
(21) Application number: 23203176.5
(22) Date of filing: 12.10.2023
(51) Int. Cl.: A61B 6/00

(54) **HOLDING ARRANGEMENT FOR AN X-RAY IMAGING DEVICE**

(71) Applicant: Koninklijke Philips N.V., 5656 AG Eindhoven (NL)
(72) Inventor: DRIES, Johan Juliana, Eindhoven (NL); VAN DER WACHT, Rogier, Eindhoven (NL); GAST, Tomas, 5656AG Eindhoven (NL)
(74) Representative: Philips Intellectual Property & Standards

(57) **Abstract**

The present invention relates to movably holding parts of an X-Ray imaging device. In order to provide further facilitated and space-saving medical X-ray imaging, a holding arrangement (10) for an X-ray imaging device is provided. The arrangement comprises a lower source robot (12) for movably holding an X-ray source and an upper detector robot (14) for movably holding an X-ray detector. The upper detector robot comprises an upper base (16), a first upper arm (18), a second upper arm (20) and a detector mount (22). The upper base comprises an upper base plate (24) and an upper base mount (26) rotatably connected to the upper base plate, wherein the upper base plate is configured to be movably mounted to a ceiling support. The first upper arm is rotatably connected to the upper base mount, the second upper arm is rotatably connected to the first upper arm and the detector mount is rotatably connected to the second upper arm. The second upper arm comprises two arm portions (28, 30) that are rotatably connected around a longitudinal upper arm axis (32) of the second upper arm. The second upper arm is having a greater length than the first upper arm.

## Description

### FIELD OF THE INVENTION

The present invention relates to movably holding parts of an X-Ray imaging device, and relates in particular to a holding arrangement for an X-ray imaging device, to a medical X-ray imaging system and to a method for X-ray imaging.

### BACKGROUND OF THE INVENTION

In medical imaging, X-ray imaging is provided for providing information about non-visible details of an object. In order to be able to provide different viewing directions, X-ray imaging systems have been developed with a movable mounted C-arm to carry and align the X-ray tube and X-ray detector. Since a C-arm is relatively bulky, X-ray systems are provided that have individual supports for source and detector. For example, WO 2020/089272 A1 describes a system with two robot arms holding the source and the detector. However, in view of a further increasing amount of equipment and required user flexibility, a demand exists for further optimizations in terms of space and user comfort.

### SUMMARY OF THE INVENTION

There may thus be a need to provide further facilitated and space-saving medical X-ray imaging.

The object of the present invention is solved by the subject-matter of the independent claims; further embodiments are incorporated in the dependent claims. It should be noted that the following described aspects of the invention apply also for the holding arrangement for an X-ray imaging device, for the medical X-ray imaging system and for the method for X-ray imaging.

According to the present invention, a holding arrangement for an X-ray imaging device is provided. The arrangement comprises a lower source robot for movably holding an X-ray source and an upper detector robot for movably holding an X-ray detector. The upper detector robot comprises an upper base, a first upper arm, a second upper arm and a detector mount. The upper base comprises an upper base plate and an upper base mount rotatably connected to the upper base plate, the latter being configured to be movably mounted to a ceiling support. The first upper arm is rotatably connected to the upper base mount, the second upper arm is rotatably connected to the first upper arm and the detector mount is rotatably connected to the second upper arm. The second upper arm comprises two arm portions that are rotatably connected around a longitudinal upper arm axis of the second upper arm. The second upper arm is having a greater length than the first upper arm.

As an effect, a maximum reduction of the arms' lengths is achieved which results in less torque occurring during operation of the imaging system. Further, the reduction of the lengths of the arms also means a reduction in building height, resulting in less movement or drive constraints, also in relation with other equipment in the vicinity.

According to an example, the length of the second upper arm is at least twice the length of the first upper arm.

According to an example, the detector mount comprises a detector mounting member and a detector retainer plate configured for fixedly attaching the X-ray detector. The detector retainer plate is connected to the detector mounting member rotatably around a detector axis configured to be aligned with a center of a detector surface of the X-ray detector. The second upper arm is connected to the detector mounting member around a mounting axis arranged perpendicular to the detector axis. The mounting axis is displaced to the detector axis by an offset that is at least a third of a diameter of the detector surface.

According to an example, the upper base mount is connected to the upper base plate rotatably around a vertical upper base rotation axis. In a first option, the vertical upper base rotation axis is aligned with a horizontal rotation axis of the rotatable connection of the first upper arm and the upper base mount. Ina second option, an offset is provided between the upper vertical base rotation axis and the horizontal rotation axis of the rotatable connection of the first upper arm and the upper base mount.

According to an example, a drive arrangement is provided to apply a driving force for achieving a relative translational or rotational movement of at least one of the group comprising the upper base plate, the upper base mount, the first upper arm, the second upper arm, the two arm portions, the detector mounting member and the detector retainer plate.

According to an example, the lower source robot comprises a lower base, a first lower arm, a second lower arm and a source mount. The first lower arm is rotatably connected to the lower base, the second lower arm is rotatably connected to the first lower arm and the source mount is rotatably connected to the second lower arm. The second lower arm comprises two arm portions that are connected rotatably around a longitudinal arm axis of the second lower arm. The lower base comprises a lower base plate and a lower base mount rotatably connected to the lower base plate, wherein the first lower arm is rotatably connected to the lower base mount; the lower base plate is movably connected to the floor support, e.g. a linear movement is provided between the floor support and the lower base plate. The second lower arm is having a greater length than the first lower arm.

According to an example, the source mount comprises a source mounting member and a source receiving segment configured for holding the X-ray source. The source receiving segment is connected to the source mounting member rotatably around a source axis to be aligned with a center of the X-ray source. The second lower arm is connected to the source mounting member around a mounting axis arranged perpendicular to the source axis. As an option, the mounting axis is displaced to the source axis by an offset that is at least approximately 25% of an outer diameter of a source housing.

According to an example, the longitudinal arm axis of the second lower arm, around which the two arm portions of the second lower arm can be rotated, is arranged with an offset to the rotatable connection of the first lower arm and the second lower arm. In an option, the offset is at least half of the diameter of the outer dimension of the second lower arm at its proximal end.

According to an example, the lower base mount is connected to the lower base plate rotatably around a vertical lower base rotation axis. In a first option, the vertical lower base rotation axis is aligned with a horizontal rotation axis of the rotatable connection of the first lower arm and the lower base mount. In a second option, an offset is provided between the vertical lower base rotation axis and the horizontal rotation axis of the rotatable connection of the first lower arm and the lower base mount.

According to the present invention, also a medical X-ray imaging system is provided. The system comprises a holding arrangement for an X-ray imaging device according to one of the preceding examples, an X-ray source mounted to the lower source robot, and an X-ray detector mounted to the upper detector robot and an object support configured to provide support to an object. The holding arrangement is configured to arrange the X-ray source and the X-ray detector for imaging of an object on the object support such that the object is between the X-ray source and the X-ray detector.

In an option, an X-ray collimator is provided to shape an X-ray beam generated by the X-ray source.

According to the present invention, also a method for X-ray imaging is provided. The method comprises the following steps:
- Movably holding an X-ray source by a lower source robot.
- Movably holding an X-ray detector by an upper detector robot. The upper detector robot comprises an upper base, a first upper arm, a second upper arm and a detector mount. The upper base comprises an upper base plate and an upper base mount rotatably connected to the upper base plate, the latter being mounted to a ceiling support. The first upper arm is rotatably connected to the upper base mount, the second upper arm is rotatably connected to the first upper arm and the detector mount is rotatably connected to the second upper arm. The second upper arm comprises two arm portions that are rotatably connected around a longitudinal upper arm axis of the second upper arm. The second upper arm is having a greater length than the first upper arm.
- Arranging an object between the X-ray source and the X-ray detector.
- Generating an X-ray beam by the X-ray source.
- Detecting X-ray radiation after at least a part of the X-ray beam has passed through the object.

According to an aspect, a first and second robot are provided that are of the same movement scheme or concepts, but due to different constraints, the concepts are adapted differently to achieve maximum overall optimization.

According to an aspect, a C-less X-ray imaging system is provided. Instead of a C-arm. Two separate robotic arms are provided as moving arrangement to hold and move the X-ray source and the X-ray detector. By providing a certain geometric scheme, the moving arrangement has a maximum degree of flexibility in terms of achieving different imaging positions and imaging view directions. The geometric scheme is based on providing a short first arm and a longer second arm e.g. for the detector robot arm, and optional also for the source robot arm, both first arm and second arm pivoting around a horizontal axis. Further adjustment is provided by a vertical axis on the ceiling mount and a further lengthwise orientation at the second arm.

According to an aspect, in an option, an offset is provided at the detector mount such that the mount is provided on an edge of the detector housing. By an additional rotation of the detector, the housing is acting as an extension of the second arm, which can then in turn be shorter.

According to an aspect, two 7-DOF (degree of freedom) robot arms are used to position the detector and the X-ray tube around the patient. The layout of the robot arms is optimized to maximally meet the desired specifications: reach required projections, allow a scan movement over at least 180°, preferably allow iso-height variation, provide enough longitudinal range, enable integrated cabling, perform movements in a limited space, i.e. to reduce chances for collisions, appealing and slim design and, for the lower robot, to remain below the tabletop as much as possible.

In principle, both the X-ray detector and tube have to be positioned in six DOFs: three positions, i.e. X/Y/Z, and three rotations, i.e. Rx, Ry, Rz. In an example, robot arms are provided that provide these six DOF on the end effector by a configuration of at least six joints where a joint can be a translational joint or a rotational joint. In an option, the optimized robot layouts have seven joints in a specific stacking order.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a holding arrangement for an X-ray imaging device, with a lower source robot and an upper detector robot. Fig. 1 also shows an example of a medical X-ray imaging system.
Fig. 2 shows an example of a positioning of the upper detector robot of the holding arrangement of Fig. 1.
Fig. 3 shows an example of another positioning of the upper detector robot of Fig. 1.
Fig. 4 shows an example of the upper detector robot of Fig. 1.
Fig. 5 shows an example of a positioning of the lower source robot of Fig. 1.
Fig. 6 shows an example of the lower source robot of Fig. 1.
Fig. 7 shows another example of the lower source robot of Fig. 1.
Fig. 8 shows an illustration of a first sequence of motion of the medical X-ray imaging system.
Fig. 9a, 9b and 9c show an illustration of a second sequence of motion of the medical X-ray imaging system.
Fig. 10 shows another example of a lower source robot in the context of a subject support.
Fig. 11 shows basic steps of an example of a method for X-ray imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

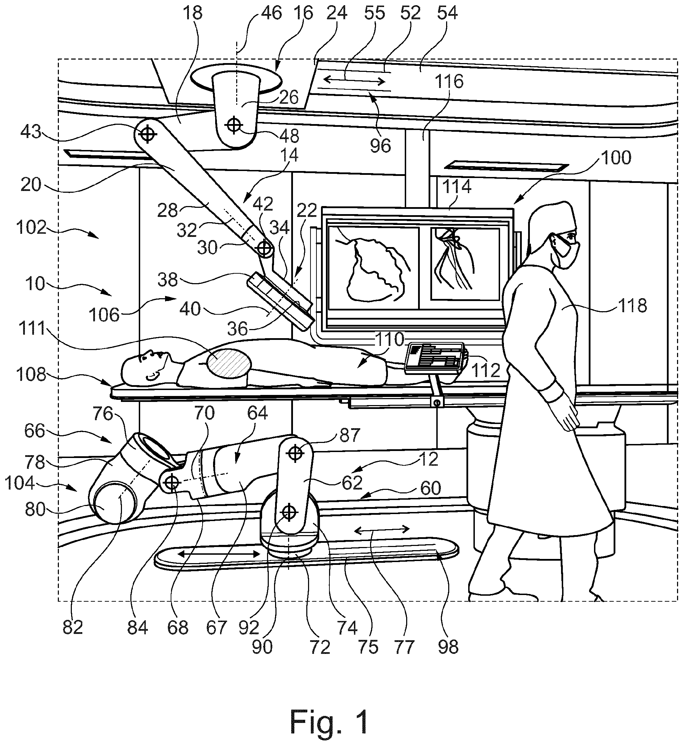

Fig. 1 schematically shows an example of a holding arrangement 10 for an X-ray imaging device. It is noted that the holding arrangement 10 is shown in the context of an example of a medical X-ray imaging system as an option. The holding arrangement 10 comprises a lower source robot 12 for movably holding an X-ray source and an upper detector robot 14 for movably holding an X-ray detector. The upper detector robot 14 comprises an upper base 16, a first upper arm 18, a second upper arm 20 and a detector mount 22. The upper base 16 comprises an upper base plate 24 and an upper base mount 26 rotatably connected to the upper base plate 24. The upper base plate 24 is configured to be movably mounted to a ceiling support. The first upper arm 18 is rotatably connected to the upper base mount 26, the second upper arm 20 is rotatably connected to the first upper arm 18 and the detector mount 22 is rotatably connected to the second upper arm 20. The second upper arm 20 comprises two arm portions 28, 30 that are rotatably connected around a longitudinal upper arm axis 32 of the second upper arm 20. The second upper arm 20 is having a greater length than the first upper arm 18.

The term "holding arrangement" relates to providing a movable mount for the X-ray source and the X-ray detector of the X-ray imaging system.

The term "lower source robot" relates to a robot for moving and holding the X-ray source. The robot is mounted to the floor and the X-ray source is mounted at the free end of the robot. The robot may have the form of an arm with several movable joints that allow a high degree of freedom of movement.

The term "upper detector robot" relates to a robot for moving and holding the X-ray detector. The robot is mounted to the ceiling and the X-ray detector is mounted at the free end of the robot. The robot may have the form of an arm with several movable joints that allow a high degree of freedom of movement.

The term "rotatably connected" relates to a connection that allows a rotating movement around a rotation axis, e.g. a pivoting movement or a turning movement.

In common medical X-ray imaging, the X-ray source is usually provided below a subject support like a subject table. The X-ray detector is arranged above the table to detect X-ray after passing through an object. The X-ray source and X-ray detector can be arranged such that they are moved in common around an iso-center. According to the present invention, the upper detector robot thus relates to a robot holding and moving an X-ray detector for medical X-ray imaging. Further, the lower detector robot relates to a robot holding and moving an X-ray source for medical X-ray imaging.

The upper detector robot 14 can also be referred to as detector robot, detector robot arm, upper robot, upper robot arm, upper movable mount, upper mount or detector mount. The upper detector robot 14 can also be referred to as second movable mount, second robot arm or second robot.

The first upper arm 18 is rotatably connected to the upper base 16 via a first upper rotation axis, and the second upper 20 arm is rotatably connected to the first upper arm 18 via a second upper rotation axis, the first upper rotation axis and the second upper rotation axis being arranged parallel to each other in a horizontal direction. The detector mount is rotatably connected to the second upper arm via a third upper rotation axis, the third upper rotation axis being arranged perpendicular to the longitudinal arm axis of the second upper arm.

The longitudinal arm axis of the second upper arm can also be referred to as longitudinal second upper arm axis.

The two arm portions of the second upper arm can also be referred to as two arms forming a longer upper beam. The two arm portions of the second upper arm can also referred to as second upper proximal arm segment and second upper distal arm segment.

The first upper arm is rotatably connected to the upper base with its proximal end. The second upper arm is rotatably connected with its proximal end to a distal end of the first upper arm. The detector mount is rotatably connected to a distal end of the second upper arm.

The proximal end of the first upper arm can also be referred to as first upper arm proximal end. The distal end of the first upper arm can also be referred to as first upper arm distal end. The proximal end of the second upper arm can also be referred to as second upper arm proximal end. The distal end of the second upper arm can also be referred to as second upper arm distal end.

In an option, the upper base plate is configured to be movably mounted to the ceiling support. The base plate can move linearly with respect to the ceiling support.

In an option of the example of Fig. 1, the length of the second upper arm is at least twice the length of the first upper arm.

In an example, a first design is provided with a first upper arm length of 470 mm (joint axis to joint axis) and a second upper arm length of 880 mm. In another robot layout, the first upper arm length is even longer. In a further example, the first and second arm lengths are 550 mm and 750 mm. In another example, 650 mm and 1050 mm are provided. In a still further example, 630 mm and 970 mm are provided.

As indicated above, Fig. 1 also shows an example of a medical X-ray imaging system 100 as an option. The system 100 comprises a holding arrangement 102 for an X-ray imaging device according to one of the preceding and following examples. The system 100 also comprises an X-ray source 104 mounted to the lower source robot 12 and an X-ray detector 106 mounted to the upper detector robot 14. The system 100 further comprises an object support 108 configured to provide support to an object 110 with a region of interest 111 to be imaged by the X-ray imaging system 100. The holding arrangement 102 is configured to arrange the X-ray source and the X-ray detector for imaging of an object on the object support such that the object is between the X-ray source and the X-ray detector.

In Fig. 1, a control panel or user interface 112 is provided in the vicinity of the subject support. Further, a display arrangement 114 is provided in the background. The display arrangement 114 can be suspended from the ceiling by a movable mount 116. A user 118 is shown in the right foreground section.

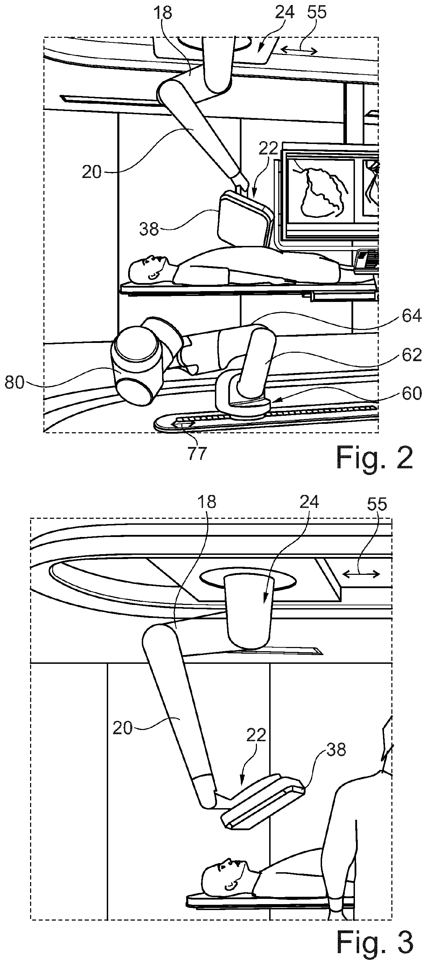

Fig. 2 shows an example of a positioning of the upper detector robot 14 of the holding arrangement 10 of Fig. 1. The viewing direction can be adjusted to the respective demands in a facilitated manner.

Fig. 3 shows another example of a positioning of the upper detector 14 robot of Fig. 1 with the imaging device having another viewing direction.

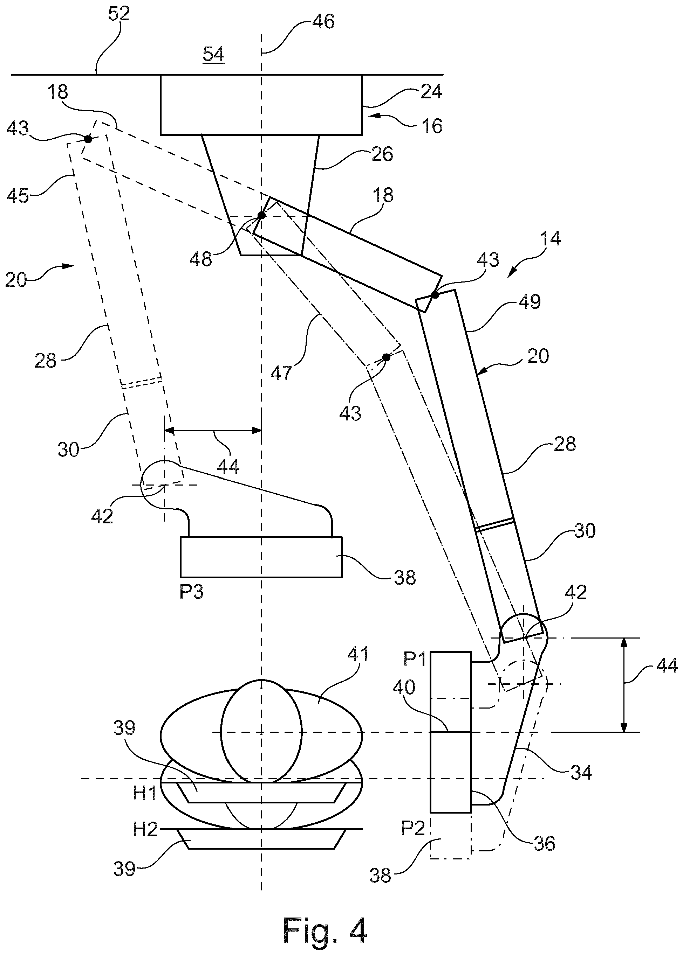

Fig. 4 shows another example of the upper detector robot of Fig. 1.

It is noted that the drawings in the figures show the X-ray detector and the X-ray source mounted to the respective upper detector robot and lower source robot. This is referred to as the medical X-ray imaging system. However, it is also provided the upper detector robot and the lower source robot without the X-ray detector and X-ray source. This is referred to as the holding arrangement for an X-ray imaging device, which can also be referred to as holding arrangement for a medical X-ray imaging system.

For the X-ray imaging system, as indicated below, an X-ray collimator to shape an X-ray beam generated by the X-ray source is provided in an option.

In an option, e.g. shown as example in Fig. 4, the detector mount 22 comprises a detector mounting member 34 and a detector retainer plate 36 configured for fixedly attaching an X-ray detector 38. The detector retainer plate 36 is connected to the detector mounting member 34 rotatably around a detector axis 40 configured to be aligned with a center of a detector surface of the X-ray detector. The second upper arm 20 is connected to the detector mounting member 34 around a mounting axis 42 arranged perpendicular to the detector axis. The mounting axis 42 is displaced to the detector axis 40 by an offset 44 (see Fig. 4) that is at least a third of a diameter of the detector surface.

The second upper arm 20 is connected to the first lower arm 18 around a horizontal axis 43.

The offset in combination with the combination of two perpendicular rotation axes and the longitudinal rotation within the second arms allows to have a reduced length of the second arm as compared to a version with non-offset mount of the detector.

In an example, the mounting axis is displaced such that is arranged outside the detector surface.

The mounting axis can also be referred to as detector mounting axis.

The detector retainer plate can also be referred to as retainer plate.

In an option, the offset is at least half of the diameter of the detector surface.

In an option, the offset is provided such that the mounting axis is displaced at least half the width of a detector housing.

In an option, a first linear movement of the base plate with respect to the ceiling is provided. This is a valuable addition of the robot movement-axis layout.

Due to the offset, an extended viewing range is possible, while still minimizing the length of the robot arms.

In an example, the upper base mount 26 is connected to the upper base plate 24 rotatably around a vertical upper base rotation axis 46.

In a first option, the vertical upper base rotation axis is aligned with a horizontal rotation axis 48 of the rotatable connection of the first upper arm and the upper base mount. In a second option, an offset 50 is provided between the upper vertical base rotation axis and the horizontal rotation axis of the rotatable connection of the first upper arm and the upper base mount.

In an example, the upper base plate is slidably mounted to an upper rail arrangement 52 configured to be fixed on a ceiling 54. The translational movement is indicated with a first double arrow 55.

In Fig. 4, a first positioning of the upper detector robot 14 is indicated with straight lines 49; a second positioning of the upper detector robot 14 is indicated with first broken lines 47 and a third positioning of the upper detector robot 14 is indicated with second broken lines 45. These different positions can take place in the plane of the drawing but also in other planes rotated around a vertical axis.

As can be seen, with the first positioning of the upper detector robot 14, a first position P1 of the detector is achieved lateral to a subject 41 on a support 39. The support 39 is arranged in a first height H1.

As can be seen further, with the second positioning of the upper detector robot 14, a second position P2 of the detector is achieved lateral to the subject 41 on the support 39. The support 39 is arranged in a second height H2.

As can also be seen, with the third positioning of the upper detector robot 14, a third position P3 of the detector is achieved above the subject 41 on the support 39.

In another example, a drive arrangement (not shown in detail) is provided to apply a driving force for achieving a relative translational or rotational movement of at least one of the group comprising the upper base plate, the upper base mount, the first upper arm, the second upper arm, the two arm portions, the detector mounting member and the detector retainer plate.

For example, the drive arrangement is provided to apply a driving force for achieving a relative rotational movement of:
- the upper base plate and the upper base mount;
- the upper base mount and the first upper arm;
- the first upper arm and the second upper arm;
- the two arm portions of the second upper arm;
- the second upper arm and the detector mounting member; and
- the detector mounting member and the detector retainer plate.

For example, the drive arrangement is provided to apply a driving force for achieving a relative linear movement of:
- the ceiling support and the upper base plate.

In an example, the second arm has a smooth outer contour with a decreasing outer diameter from its proximal end to its distal end.

In an example, the smooth outer contour is achieved by an arm housing that also covers the portions of the rotatable connection. The term smooth relates to a contour free of steps, protrusions or dents.

In an option, the first arm has a smooth outer contour with a decreasing outer diameter from its proximal end to its distal end.

In a further option, the upper base mount has a smooth outer contour with a decreasing outer diameter from its proximal end to its distal end.

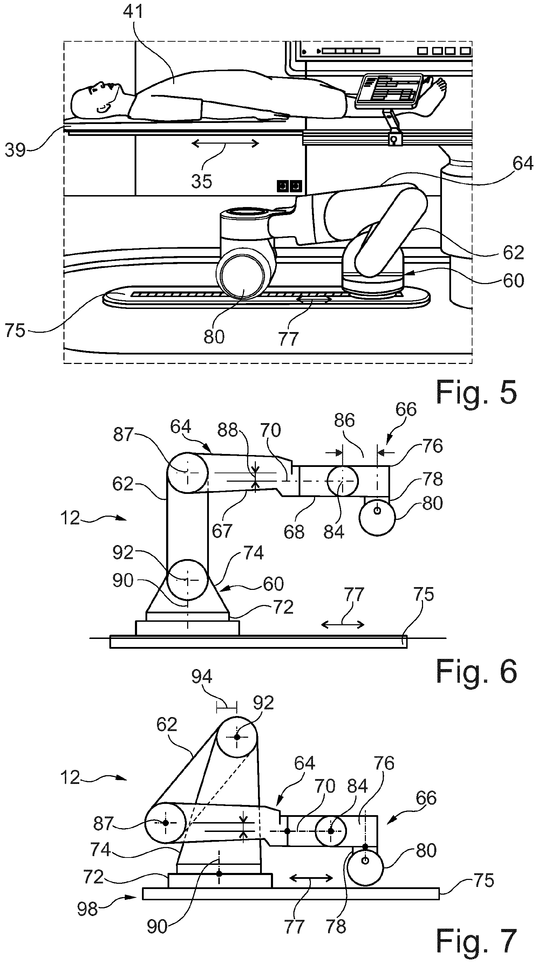

Fig. 5 shows an example of a positioning of the lower source robot 12 of Fig. 1. The subject support may be translatable in a longitudinal direction, as indicated with a third double arrow 35.

Fig. 6 shows an example of the lower source robot of Fig. 1.

Fig. 7 shows another example of the lower source robot of Fig. 1.

Also with reference to Fig. 1, in an example shown in Fig. 6 and Fig. 7, the lower source robot 12 comprises a lower base 60, a first lower arm 62, a second lower arm 64 and a source mount 66. The first lower arm 62 is rotatably connected to the lower base 60, the second lower arm 64 is rotatably connected to the first lower arm 62 and the source mount 66 is rotatably connected to the second lower arm 64. The second lower arm 64 comprises two arm portions 67, 68 that are connected rotatably around a longitudinal arm axis 70 of the second lower arm 64. The lower base 60 comprises a lower base plate 72 and a lower base mount 74 rotatably connected to the lower base plate 72. The first lower arm 62 is rotatably connected to the lower base mount 74; the lower base plate 72 is movably connected to a floor support, e.g. on a floor support rail 75. The translational movement is indicated with a second double arrow 77. The second lower arm 64 is having a greater length than the first lower arm 62.

The lower source robot can also be referred to as source robot, source robot arm, lower robot, lower robot arm, lower movable mount, lower mount or source mount. The lower source robot can also be referred to as first movable mount, first robot arm or first robot.

The first lower arm is rotatably connected to the lower base via a first lower rotation axis, and the second lower arm is rotatably connected to the first lower arm via a second lower rotation axis, the first lower rotation axis and the second lower rotation axis being arranged parallel to each other in a horizontal direction. The source mount is rotatably connected to the second lower arm via a third lower rotation axis, the third lower rotation axis being arranged perpendicular to the longitudinal arm axis of the second lower arm.

In an option, a fist linear movement of the first lower arm is provided.

The longitudinal arm axis of the second lower arm can also be referred to as longitudinal second lower arm axis.

The two arm portions of the second lower arm can also be referred to as two arms forming a longer lower beam. The two arm portions of the second lower arm can also referred to as second lower proximal arm segment and second lower distal arm segment.

The first lower arm is rotatably connected to the lower base with its proximal end. The second lower arm is rotatably connected with its proximal end to a distal end of the first lower arm. The source mount is rotatably connected to a distal end of the second lower arm.

The proximal end of the first lower arm can also be referred to as first lower arm proximal end. The distal end of the first lower arm can also be referred to as first lower arm distal end. The proximal end of the second lower arm can also be referred to as second lower arm proximal end. The distal end of the second lower arm can also be referred to as second lower arm distal end.

In an option, the length of the second lower arm is at least twice the length of the first lower arm.

In further examples, as arm lengths in the robot design of the upper detector robot, an example of the first upper arm has a length of approximately 315 mm, while an example of the second upper arm has a length of approximately 670 mm.

In further examples, as arm lengths in the robot design of the upper detector robot, an example of the first upper arm has a length of approximately 470 mm, while an example of the first arm portion of the second upper arm has a length of approximately 400 mm and the an example of the first arm portion of the second upper arm has a length of approximately 480 mm.

In an example, the second lower arm is even shorter than the first lower arm.

For example, a combination with an offset between the base mount rotation and the first lower arm rotation is provided, thus enabling to reduce the length of the second lower arm.

In an option, the length of the first lower arm is provided such that an upper edge of the first lower arm when in its upright position is below a subject support table in a working height. This ensures that the lower robot arm always stays below the subject support with its central base part.

In an option, the lower source robot stays below the tabletop as much as possible. As an example, the second arm, e.g. with its combination of two arm segments uses space on both sides of the base: a smaller part on one side when oriented in a horizontal manner and a larger part at the other side of the base, e.g. between the base and the X-ray tube. As a result, the largest part of the system, i.e. the second lower arm stays below the tabletop.

In an example, see also Fig. 6, the source mount 66 comprises a source mounting member 76 and a source receiving segment 78 configured for holding an X-ray source 80. The source receiving segment 78 is connected to the source mounting member 76 rotatably around a source axis 82 to be aligned with a center of the X-ray source 80. The second lower arm 64 is connected to the source mounting member 76 around a mounting axis 84 arranged perpendicular to the source axis 82. In an option, the mounting axis 84 is displaced to the source axis 82 by an offset 86 (see Fig. 6) that is at least approximately 25% of an outer diameter of a source housing.

It is noted that according to an option, also an X-ray collimator is provided, but not shown in detail. The X-ray collimator is configured to shape an X-ray beam generated by the X-ray source.

The second lower arm 64 is mounted to the first lower arm 62 around a horizontal axis 87.

In an option, the X-ray source arm is rather long. For example, it is at least half of the diameter of the collimator which is placed above the X-ray source.

In an example, the mounting axis is displaced to the source axis by an offset that is at least approximately 25% or30% of the outer diameter of the source housing, e.g. approximately 50% of the outer diameter of the source housing.

In an example, the mounting axis is displaced by an offset that is at least the outer diameter of the source housing.

The mounting axis can also be referred to as source mounting axis.

In an example, the longitudinal arm axis 70 of the second lower arm 64, around which the two arm portions of the second lower arm 64 can be rotated, is arranged with an offset 88 to the rotatable connection, i.e. the horizontal axis 87, of the first lower arm and the second lower arm. In an option, the offset 88 is at least half of the diameter of the outer dimension of the second lower arm at its proximal end.

In an example, the lower base mount 74 is connected to the lower base plate 72 rotatably around a vertical lower base rotation axis 90.

In a first option, shown in Fig. 6, the vertical lower base rotation axis 90 is aligned with a horizontal rotation axis 92 of the rotatable connection of the first lower arm 62 and the lower base mount 74.

In a second option, shown in Fig. 7, an offset 94 is provided between the vertical lower base rotation axis and the horizontal rotation axis of the rotatable connection of the first lower arm and the lower base mount.

In a further option, the lower base plate is slidably mounted to a lower rail arrangement configured to be fixed on a floor.

In a further option, a drive arrangement is provided to apply a driving force for achieving a relative translational or rotational movement of at least one of the group comprising the lower base plate, the lower base mount, the lower upper arm, the second lower arm, the two arm portions, the source mounting member and the source receiving segment.

For example, the drive arrangement is provided to apply a driving force for achieving a relative rotational movement of:
- the lower base plate and the lower base mount;
- the lower base mount and the first lower arm;
- the first lower arm and the second lower arm;
- the two arm portions of the second lower arm;
- the second lower arm and the source mounting member; and
- the source mounting member and the source receiving segment.

For example, the drive arrangement is provided to apply a driving force for achieving a relative linear movement of:
- the floor support and the lower base plate.

In an example, also shown as option in Fig. 1, for further increase of range of imaging, the upper base is configured to be slidably mounted to an upper rail arrangement 96.

In another example, in addition or alternatively, shown as another option in Fig. 1, the lower base is configured to be slidably mounted to a lower rail 98 arrangement.

In an option, a drive is provided to apply a driving force for achieving a sliding, i.e. translational movement of the upper base along the upper rail arrangement.

In a further option, a drive is provided to apply a driving force for achieving a sliding, i.e. translational movement of the lower base along the lower rail arrangement.

In an example, not shown in detail, a control unit is provided to control a coordinated movement of the first movable mount and the second movable mount.

In an example, the X-ray detector is mounted to the detector mount of the upper source robot. The X-ray source is mounted to the source mount of the lower source robot.

In an example, the X-ray detector is mounted to the detector retainer plate of the detector mount. The X-ray source is mounted to the source receiving segment of the source mount.

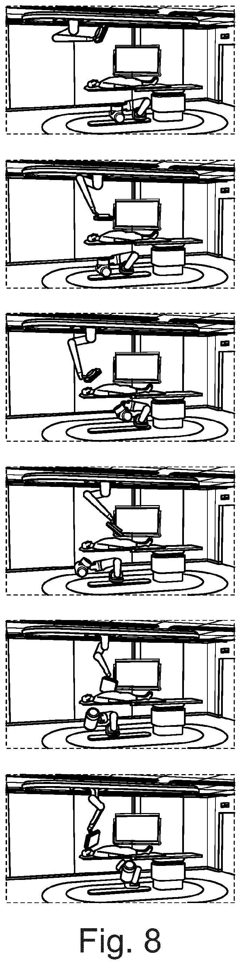

Fig. 8 shows an illustration of a first sequence of motion of the medical X-ray imaging system. The upper frame shows a starting or parting position. The upper detector robot 14 is arranged such that the detector is moved upwards below the ceiling. The lower source robot 14 is arranged such that the source and its holding arms are completely arranged below the subject support. The further frames from the top show several imaging positions. As can be seen, the detector and the source can be moved in common to a large variety of positions allowing different viewing directions.

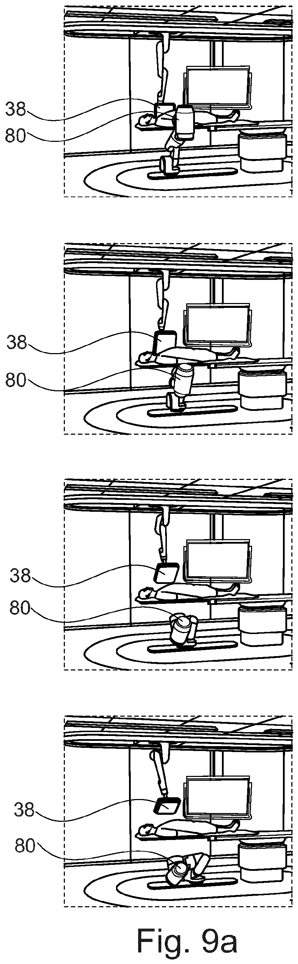

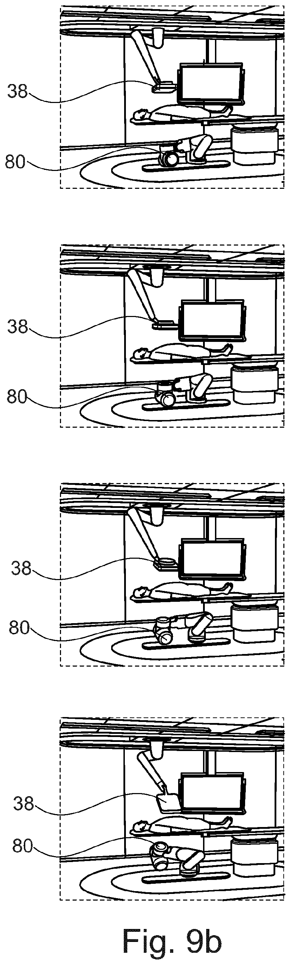

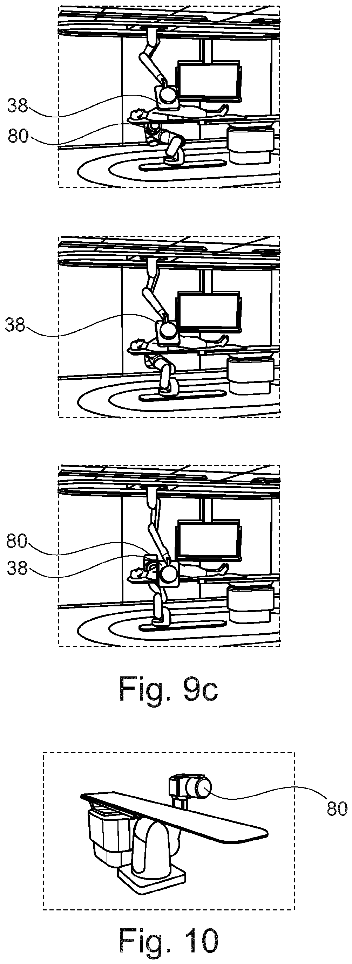

Fig. 9a, 9b and 9c show an illustration of a second sequence of motion of the medical X-ray imaging system. In Fig. 9a, a viewing direction is changed from horizontal to inclined. In Fig. 9b, the viewing direction is changed from inclined to vertical. In Fig. 9c, a viewing direction is changed from inclined to horizontal. Thus, a complete trajectory of 180° is provided.

Fig. 10 shows another example of the lower source robot 12 in the context of a subject support. As can be seen, the source 80 can be moved to a position above the table height, while the holding arms are arranged below the subject support to a large extent. The constraints for the users in terms of space occupied by the holding mechanism for the X-ray imaging is kept to a minimum.

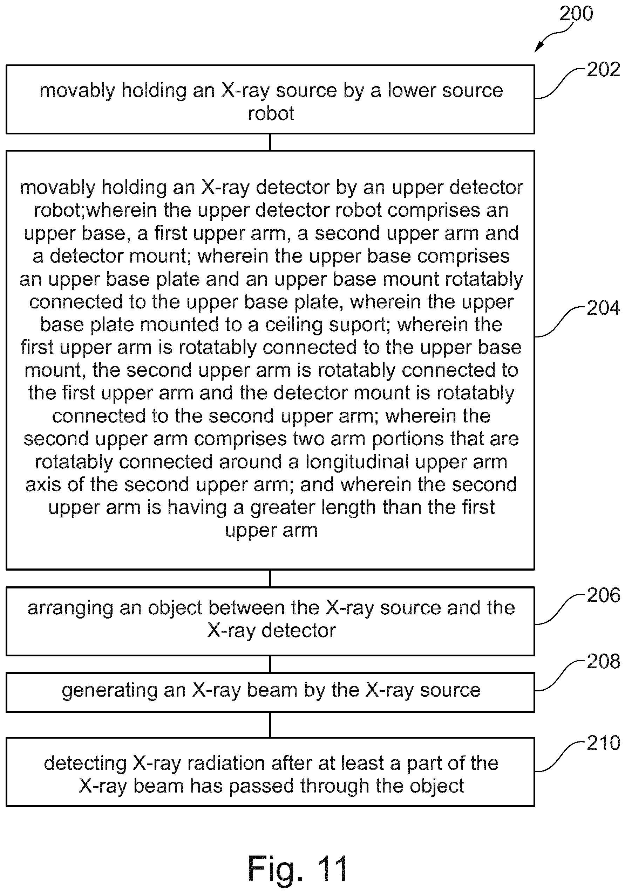

Fig. 11 shows basic steps of an example of a method 200 for X-ray imaging. The method 200 comprises the following steps:
- In a first step 202, an X-ray source is movably hold by a lower source robot.
- In a second step 204, an X-ray detector is movably hold by an upper detector robot. The upper detector robot comprises an upper base, a first upper arm, a second upper arm and a detector mount. The upper base comprises an upper base plate and an upper base mount rotatably connected to the upper base plate. The upper base plate is mounted to a ceiling support. The first upper arm is rotatably connected to the upper base mount, the second upper arm is rotatably connected to the first upper arm and the detector mount is rotatably connected to the second upper arm. The second upper arm comprises two arm portions that are rotatably connected around a longitudinal upper arm axis of the second upper arm. The second upper arm is having a greater length than the first upper arm.
- In a third step 206, an object is arranged between the X-ray source and the X-ray detector;
- In a fourth step 208, an X-ray beam is generated by the X-ray source.
- In a fifth step 210, X-ray radiation is detected after at least a part of the X-ray beam has passed through the object.

An optimized robot layout for both robots is provided that are part of a C-less system configuration, i.e. a configuration without a C-arm. Briefly said, C-less represents the idea where in a fixed X-ray system for interventional or hybrid procedures, the C-arm, also known as C-arc, is not present anymore. Two robot arms are used to manipulate, i.e. handle, the X-ray tube and the X-ray detector in a coordinated fashion. For both robot arms, optimized robot configurations are provided. The optimization takes into account the application requirements, the available space and the presence of staff and other equipment in the examination room.

As an advantage, removing the C-arm from the operational room frees up a lot of space and improves patient access during the procedures. As an example, if needed an additional staff member can be standing close to the patient, e.g. during more complex hybrid procedures. Without the C-arm, there is basically unlimited access to the patient table and patient during the patient preparation. This helps in reducing preparation time. Without the C-arm, the room setup is much easier and requires less intensive planning, which is often necessary when a C-arm is present that requires the staff to prepare the intended procedure very well to plan where the C-arm will be placed and where to position the required staff and other equipment.

As a further advantage, for hybrid procedures that involve additional robots, the openness due to minimized space occupation by the X-ray imaging system of the present solution also provides more space.

The provided setup also allows to obtain images from steeper projections, e.g. during cardiac procedures. When using a C-arc, collisions by the detector and C-arc with the patient or tabletop may prevent these. However, using the flexibility of separate robotic suspensions will enable these steeper projections, e.g. an increase up to 10° or even 15°.

The robotic solution is very flexible enabling a wider range of 3D-scan trajectories and will have less interference, in particular useful when the staff wants to perform 3D-scans. Less occupied space also means less interferences with the tabletop and anaesthesia or ECG equipment. Also, the C-arc typical limitation of the physical range of the detector travel is omitted.

Unlike C-arc systems with a fixed iso-center height, i.e. height of the rotation point for rotations and angulations, the flexibility of the robotic system allows a flexible iso-center height which implies that much more physicians will be able to position the patient at the optimal ergonomic height.

The presently provided C-less robots are also beneficial regarding building structure related aspects: Compared to C-arm solutions, a weight reduction of more than 50% and more is provided, which will enable much easier ceiling interfaces. This will save significant time and money for the hospital in view of room preparation constructions to be performed by the hospital.

Next to the weight, also the required footprint of the solution within the operational room is much smaller, e.g. a reduction by a third up to a half which enables the hospitals to make more efficient use of their available space, or reduce the hassle to create bigger examination rooms.

In terms of an environmental, or space-economic design, the present solution has a big impact on the three pillars: weight reduction, longer lifetime of the industrial robot technology and possible reuse of the robots in new systems. As an example, the robots and other modules are designed for circularity purposes.

In an example, both robot arms can be moved in longitudinal direction by placing them on a translational joint, e.g. 'longitudinal carrier' on 'longitudinal rails'. Both robot arms themselves contain six rotational joints. All these joints are used to enable the applicational relevant movements, like e.g. rotations around the `region of interest'. The X-ray tube and X-ray detector need to be properly aligned when X-ray images are acquired. Note that the position of the `region of interest' can change throughout the procedure.

In an example, the joints of both robots have a specific stacking order (FD = detector robot, TR = tube robot). In the following, the two portions of the second arm are referred to as "arm3" and "arm4":
- translational joint in Y-direction: FD_baseY & TR_baseY
- rotational joint around vertical (Z-)axis: FD_baseRz & TR_baseRz
- rotational joint around horizontal (X-)axis: FD_arm1Rx & TR_arm1Rx
- rotational joint around horizontal (X-)axis: FD_arm2Rx & TR_arm2Rx
- rotational joint around axial axis: FD_arm3Rz & TR_arm3Ry
- rotational joint around horizontal (X-)axis: FD_arm4Rx & TR_arm4Rx
- rotational joint around vertical (Z-)axis: FD_detRz & TR_tubeRz

In an option, an optimized setup for the detector robot is provided by:
- Stacking order of axes as mentioned above.
- The length of arm 1 is as short as possible to reduce the chance of collisions when the whole robot rotates along the vertical axis. This helps in avoiding collisions with monitor, monitor ceiling suspension, ceiling suspended X-ray shield(s), ceiling suspended equipment booms or other ceiling suspended tools.
- The presence of the axial rotational joint (between arm2 and arm3) enables the +180° scan and also enables an immediate movement-start of the detector in all directions. As physicians might want to perform an angulation or rotation of the X-ray beam around the region of interest, it is avoided the need to rotate the whole robot about 90° before the X-ray beam rotation movement itself can be initiated.
- The length of arm 1, arm2, arm3 combined with the FD rotation offset is optimized so that the detector can be placed next to the patient, e.g. for lateral projections, but also beyond in case the patient height is lowered and/or to reach rotation angles beyond 90°, e.g. up to 100° or more. The length is also such that the `elbow', i.e. joint between arm1 and arm2, is not elevated too much when the detector is above the patient, because that will cause collisions with the ceiling and/or longitudinal carriage.
- The human-like design where dimensions become smaller as you get closer to the end-effector. The lower parts of the robot-arm, i.e. arm2, arm3 and below, are within the viewing area of the physician. The smaller the appearance or diameter of these parts, the less they obstruct viewing the items or persons behind the robot-arm.

In an option, an optimized setup for the X-ray source robot is provided by:
- Stacking order of axes as mentioned above.
- The length of arm 1, including the construction of the longitudinal rails and the base of the robot, is not too large such that the patient table can be placed in the lowest position. This allows the patient to step on or off the table easily.
- The length of arm 1, arm2, arm3 in combination with the tube rotation offset has to be sufficient to place the X-ray source next to the patient at sufficient direction to allow lateral X-ray projections and beyond, also in case the patient height is elevated.
- The robot arm is designed such that it maximally stays below the tabletop, e.g. having a width of 500 mm, when a physician moves to different X-ray projection angles. This is beneficial to avoid collisions of the robot with the legs of the staff standing close to the patient and also with carts or other equipment next to the patient table.
- The space occupied by the robot, e.g. carriage, base, arm 1, arm2 and arm3, is not only limited in height, but also limited in length and width. The width must be smaller than the width of the tabletop. The length must also be as small as possible, such that the `occupied distance' between the X-ray tube and the table base is not too large. Also in the reverse position of the X-ray tube robot, the occupied distance towards the head-end should not be too large.

In an option, as an additional or alternative optimization, it is provided: The rotation point of arm1 can be positioned above the rotation point of arm2. It also shows that the various rotation points do not necessarily have to be in-line with each other. For example, there is a horizontal offset between 'arm1 rotation' and the `base rotation'. There is also a vertical offset between 'arm2 rotation' and 'arm3 rotation'.

The solutions provided in the present context are suitable for interventional, hybrid and surgical therapies on fixed X-ray systems. In an example, a monoplane system is provided. In a further example, a biplane system is provided: one instantiation could be the combination of a C-less system with a two-robot system, where a suspended C-arc could act as the lateral channel.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A holding arrangement (10) for an X-ray imaging device, the arrangement comprising:
- a lower source robot (12) for movably holding an X-ray source; and
- an upper detector robot (14) for movably holding an X-ray detector;
wherein the upper detector robot comprises an upper base (16), a first upper arm (18), a second upper arm (20) and a detector mount (22);
wherein the upper base comprises an upper base plate (24) and an upper base mount (26) rotatably connected to the upper base plate, wherein the upper base plate is configured to be movably mounted to a ceiling support;
wherein the first upper arm is rotatably connected to the upper base mount, the second upper arm is rotatably connected to the first upper arm and the detector mount is rotatably connected to the second upper arm;
wherein the second upper arm comprises two arm portions (28, 30) that are rotatably connected around a longitudinal upper arm axis (32) of the second upper arm; and
wherein the second upper arm is having a greater length than the first upper arm.

2. Arrangement according to claim 1, wherein the length of the second upper arm is at least twice the length of the first upper arm.

3. Arrangement according to claim 1 or 2, wherein the detector mount comprises a detector mounting member (34) and a detector retainer plate (36) configured for fixedly attaching the X-ray detector;
wherein the detector retainer plate is connected to the detector mounting member rotatably around a detector axis (40) configured to be aligned with a center of a detector surface of the X-ray detector;
wherein the second upper arm is connected to the detector mounting member around a mounting axis (42) arranged perpendicular to the detector axis; and
wherein the mounting axis is displaced to the detector axis by an offset (44) that is at least a third of a diameter of the detector surface.

4. Arrangement according to one of the claims 1 to 3, wherein the upper base mount is connected to the upper base plate rotatably around a vertical upper base rotation axis (46); and
wherein:
i) the vertical upper base rotation axis is aligned with a horizontal rotation axis of the rotatable connection of the first upper arm and the upper base mount; or
ii) an offset (50) is provided between the upper vertical base rotation axis and the horizontal rotation axis of the rotatable connection of the first upper arm and the upper base mount.

5. Arrangement according to one of the preceding claims, wherein the upper base plate is slidably mounted to an upper rail (52) arrangement configured to be fixed on a ceiling.

6. Arrangement according to one of the preceding claims, wherein a drive arrangement is provided to apply a driving force for achieving a relative translational or rotational movement of at least one of the group comprising the upper base plate, the upper base mount, the first upper arm, the second upper arm, the two arm portions, the detector mounting member and the detector retainer plate.

7. Arrangement according to one of the preceding claims, wherein the second arm has a smooth outer contour with a decreasing outer diameter from its proximal end to its distal end.

8. Arrangement according to one of the preceding claims, wherein the lower source robot comprises a lower base (60), a first lower arm (62), a second lower arm (64) and a source mount (66);
wherein the first lower arm is rotatably connected to the lower base, the second lower arm is rotatably connected to the first lower arm and the source mount is rotatably connected to the second lower arm;
wherein the second lower arm comprises two arm portions (67, 68) that are connected rotatably around a longitudinal arm axis of the second lower arm;
wherein the lower base comprises a lower base plate (72) and a lower base mount (74) rotatably connected to the lower base plate, wherein the first lower arm is rotatably connected to the lower base mount; wherein the lower base plate is movably connected to the floor support; and
wherein the second lower arm is having a greater length than the first lower arm.

9. Arrangement according to claim 8, wherein the source mount comprises a source mounting member (76) and a source receiving segment (78) configured for holding the X-ray source;
wherein the source receiving segment is connected to the source mounting member rotatably around a source axis (82) to be aligned with a center of the X-ray source;
wherein the second lower arm is connected to the source mounting member around a mounting axis (84) arranged perpendicular to the source axis; and
wherein the mounting axis is displaced to the source axis by an offset that is at least approximately 25% of an outer diameter of a source housing.

10. Arrangement according to claim 8 or 9, wherein the longitudinal arm axis of the second lower arm, around which the two arm portions of the second lower arm can be rotated, is arranged with an offset (88) to the rotatable connection of the first lower arm and the second lower arm; and
wherein the offset is at least half of the diameter of the outer dimension of the second lower arm at its proximal end.

11. Arrangement according to one of claims 8 to 10, wherein the lower base mount is connected to the lower base plate rotatably around a vertical lower base rotation axis (90); and
wherein:
i) the vertical lower base rotation axis is aligned with a horizontal rotation axis of the rotatable connection of the first lower arm and the lower base mount; or
ii) an offset (94) is provided between the vertical lower base rotation axis and the horizontal rotation axis of the rotatable connection of the first lower arm and the lower base mount.

12. Arrangement according to one of the preceding claims, for further increase of range of imaging:
i) the upper base is configured to be slidably mounted to an upper rail arrangement; and/or
ii) the lower base is configured to be slidably mounted to a lower rail arrangement.

13. Arrangement according to one of the preceding claims, wherein a control unit is provided to control a coordinated movement of the first movable mount and the second movable mount.

14. A medical X-ray imaging system (100), comprising:
- a holding arrangement (102) for an X-ray imaging device according to one of the preceding claims;
- an X-ray source (104) mounted to the lower source robot, and an X-ray detector (106) mounted to the upper detector robot; and
- an object support (108) configured to provide support to an object;
wherein the holding arrangement is configured to arrange the X-ray source and the X-ray detector for imaging of an object on the object support such that the object is between the X-ray source and the X-ray detector.

15. A method (200) for X-ray imaging, comprising the following steps:
- movably holding (202) an X-ray source by a lower source robot;
- movably holding (204) an X-ray detector by an upper detector robot; wherein the upper detector robot comprises an upper base, a first upper arm, a second upper arm and a detector mount; wherein the upper base comprises an upper base plate and an upper base mount rotatably connected to the upper base plate, wherein the upper base plate mounted to a ceiling support; wherein the first upper arm is rotatably connected to the upper base mount, the second upper arm is rotatably connected to the first upper arm and the detector mount is rotatably connected to the second upper arm; wherein the second upper arm comprises two arm portions that are rotatably connected around a longitudinal upper arm axis of the second upper arm; and wherein the second upper arm is having a greater length than the first upper arm;
- arranging (206) an object between the X-ray source and the X-ray detector;
- generating (208) an X-ray beam by the X-ray source; and
- detecting (210) X-ray radiation after at least a part of the X-ray beam has passed through the object.
